# EUROPEAN PATENT SPECIFICATION

(11) **EP 2 501 366 B1**
(45) Date of publication and mention of the grant of the patent: **26.07.2017**
(21) Application number: 10808469.0
(22) Date of filing: 17.03.2010
(51) Int. Cl.: A61K 9/16, A61K 36/60, A61K 33/38, A61P 31/12

(54) **ANTI-VIRAL COMPOSITION**
ANTIVIRUSZUSAMMENSETZUNG
COMPOSITION ANTIVIRALE

(30) Priority: 14.08.2009 US 541856
(43) Date of publication of application: 26.09.2012
(73) Proprietor: Kiani, Iraj E., Huntington Beach, CA 92648 (US)
(72) Inventor: Kiani, Iraj E., Huntington Beach, CA 92648 (US)
(74) Representative: Eisenführ Speiser
(86) International application number: PCT/US2010/027680
(87) International publication number: WO 2011/019414

(56) References cited:
- US-A1- 2006 182 813
- US-B1- 7 700 137
- BOHLOOLI SHAHAB ET AL: "Comparative study of fig tree efficacy in the treatment of common warts (Verruca vulgaris) vs. cryotherapy.", INTERNATIONAL JOURNAL OF DERMATOLOGY MAY 2007, vol. 46, no. 5, May 2007 (2007-05), pages 524-526, XP002696287, ISSN: 0011-9059
- EBRAHIMI ET AL.: 'Efficacy of 10% silver nitrate solution in the treatment of common warts: a placebo-controlled, randomized, clinical trial.' INT J DERMATOL. vol. 46, no. 2, 2007, pages 215 - 217, XP008161446
- 'ZERRIN.' UNRIPE FIG JAM, [Online] 20 June 2009, pages 1 - 18, XP008161447 Retrieved from the Internet: <URL:http://www.giverecipe.com/Tag/skin-war ts-treatment>
- FIG LEAF JUICE, [Online] 14 May 2005, pages 1 - 9, XP008162700 Retrieved from the Internet: <URL:http://ww.thriftyfun.com/tf100361.tip. html>
- HOW TO GET RID OF WARTS THE NATURAL WAY, [Online] 22 February 2009, page 4, XP008161460 Retrieved from the Internet: <URL:http://traditionalhealing.suite101.com /article.cfm/how_to_get_rid_of_warts the_natural_way>

## Description

### TECHNICAL FIELD

This invention relates to compositions for use in treating viral conditions.

### BACKGROUND

Viruses have long plagued the earth resulting in conditions ranging from simple to severe discomfort, such as that caused by warts, human papilloma virus ("HPV"), herpes, and shingles, to death such as that caused by HIV and cancer. Unlike bacterial and fungal infections, viral infections have proven difficult to treat, partly dupe to the fact that viral infections involve injection of the virus's genetic material into the host cell and partly because of the virus' ability to mutate.

As a result, a long-felt need has existed in the medical field for effectively treating viral conditions, including various categories of warts and HPV, without undesirable side effects. According to the World Health Organization, at least 630 million people worldwide are infected with HPV, and at least 75% of sexually active people in the United States contract HPV at some point in their lives. The United States Center for Disease Control further reports that, each year, there are some 6.2 million new cases of genital HPV infection in the United States alone. The American Social Health Association places the number of total cases in the United States at 40 million, and the Mayo Clinic estimates that the number is 25 million total in the United States.

Despite advances in technology and scientific research pathogens, such as viruses, bacteria, fungus, and other microorganisms, continue to cause disease, disorders, and dysfunctions. Current treatments suffer from inefficacy and/or undesirable side effects. Often, treatments that are effective for one patient are not effective for another. Thus, scientists and physicians are in continual pursuit of new treatments and drugs that are more effective, less toxic, and easily produced.

Current treatments for warts include the use of chemicals that are painful and leave unsightly scab-like coatings, liquid nitrogen that is also painful and could lead to scarring, certain laser treatments, and surgery. All of these treatments are often painful and costly and can leading to lasting, unsightly scars. After treatment, the viral condition or warts may well return, thereby subjecting the patient to pain and the inconvenience and embarrassment of ineffective treatments.

### DISCLOSURE OF INVENTION

The present invention is directed to an antiviral composition for use in the treating viral infections comprising, among other things, a silver-containing substance consisting of colloidal silver, silver nitrate, or some combination thereof, unripe fig.

### MODES OF CARRYING OUT INVENTION

The present invention is directed towards the prevention and treatment of a viral condition or disorder caused by a pathogen with a composition comprising a silver-containing substance selected from the group consisting of colloidal silver, silver nitrate, and any combination thereof, and unripe fig. Diseases, disorders, or conditions caused by such pathogens include, without limitation, cancer, warts, herpes, shingles, genital herpes, and infections caused by the human papilloma virus.

One embodiment is an antiviral composition for use in treating a viral condition, comprising a therapeutically effective amount of a silver-containing substance, which could be colloidal silver, a silver nitrate, or some combination thereof, and a therapeutically effective amount of unripe fig. The administration of silver-containing substances, such as colloidal silver, silver nitrate, or a combination thereof, in combination with unripe fig appears to have greatly superior effects and efficacy far beyond any effectiveness of either the silver-containing substances themselves or the unripe fig by itself.

The silver-containing substances may be present in an amount of anywhere from approximately 1% to approximately 75% by weight of the total composition. In another embodiment, the amount of such silver-containing substances may be approximately 1 % to approximately 25% of the composition by weight. In more typical embodiments, the amount of the silver-containing substance may be approximately 1% to approximately 55% of the composition by weight.

In certain embodiments, the antiviral composition comprises colloidal silver, silver nitrate, or some combination thereof, and natural herbs derived from the woody trees, shrubs, and/or vines of unripe figs from the Moraceae family, or any combination thereof. Furthermore, in some embodiments, the composition may further comprise red or fig vinegar, or the composition may be fermented.

This natural herb, unripe fig, may be present in an amount of anywhere from approximately 1% to approximately 25% by weight of the total composition. In another embodiment, the amount of such natural herb may be approximately 1% to approximately 75% of the composition by weight. In more typical embodiments, the amount of the natural herb may be approximately 1% to approximately 45% of the composition by weight.

Also disclosed is a method of treating a viral condition, comprising administering a therapeutically effective amount of a silver-containing substance, such as typically colloidal silver, silver nitrate, or combination thereof, and administering a therapeutically effective amount of unripe fig, whereby the viral condition is ameliorated. In certain embodiments, a viral condition may be treated by administering a therapeutically effective amount of the above-mentioned silver-containing component or components and a therapeutically effective amount of unripe fig.

The administration step may be any method of drug delivery, for example, topical application, oral administration, patch application, rinse solution, subcutaneous injection, or the like. Most typically, the composition is administered by topical application. Topical application includes any application resulting in administration of the composition to the surface of the skin. Generally, one or a few drops (depends on the size of lesions) of the composition are sufficient for small treatment areas of 1 millimeter diameter or less. Preferably, a sufficient amount of the composition should be applied such that any sores, lesions, neoplasm, or tumors are sufficiently covered.

In embodiments administered topically, the composition may further comprise a cream, gel, ointment, tablet, capsule, patch, and the like to prolong the contact between the active ingredients and the point of treatment on the skin.

In many embodiments, the composition for use according to the present invention may be administered once every other day. In other embodiments, the composition may be administered once per week or more. In still other embodiments, the composition may be administered once or more a day.

### EXAMPLES

A male subject suffered from a plantar wart on the bottom of his foot for thirty (30) years. He had seen numerous doctors, tried many unsuccessful attempts to treat the wart, including numerous over-the-counter and doctor-prescribed treatments, as well as attempting to freeze the wart, and in frustration, twice attempting to physically remove the wart himself. The subject then was treated topically with a composition comprising 30% by weight of total composition of 200 ppm the silver-containing substance and 70% by weight of total composition of a combination of unripe fig. The subject was treated once per week for four weeks. After four weeks of this completely non-invasive treatment, the plantar wart had entirely disappeared with no residual pain, no burning, no scarring or open wounds, and last but not the least, **no recurrences after more than 2 and a half years and counting.**

In another example, an attorney came to me with his eight-year-old son who had had an aggressive and tenacious plantar wart for nearly two years. The boy had been to numerous other doctors and dermatologists and the family had even considered surgery in their frustration, disappointment, and concern for the boy's wellbeing. The wart, located on the bottom of the boy's foot, was keeping him from walking normally and from numerous other normal childhood activities. He was then treated with an anti-viral composition in keeping with the present invention, administering the composition topically just once a week. The treatment is entirely painless and non-invasive. In less than two months, the anti-viral composition had eliminated all traces of the wart. Again, this was nearly two and a half years ago, and the wart has never returned.

As yet another example, another patient had had a wart on her left cheek since she was a child. After topically administering once a week for four weeks another composition in keeping with the present invention, the wart had completely disappeared. This was in August 2007, and the wart has never returned.

In yet other treatments, positive results were also seen with other concentration of silver-containing substance, including 30% by weight of 30 ppm. These and many more testimonials from patients, including many professionals and the attorney mentioned above, as well as documented evidence of these highly successful and unexpected results, are available upon request.

## Claims

1. Antiviral composition for use in the treatment of a viral condition, comprising:
(a) a therapeutically effective amount of a silver-containing substance selected from the group consisting of colloidal silver, silver nitrate, and any combination thereof; and
(b) a therapeutically effective amount of unripe fig;
(c) wherein the antiviral composition is suitable for treatment of the viral condition in a subject in need thereof.

2. The antiviral composition for use according to claim 1, wherein the silver-containing substance or silver nitrate is present in an amount between approximately 1% to approximately 75% by weight of the total antiviral composition.

3. The antiviral composition for use according to claim 1, wherein the unripe fig is present in an amount between approximately 25% and approximately 99% by weight of the total antiviral composition.

4. The antiviral composition for use according to claim 1, wherein the silver-containing substance is present in an amount between approximately 1% to approximately 55% by weight of the total antiviral composition.

5. The antiviral composition for use according to claim 1, wherein the unripe fig is present in an amount between approximately 45% and approximately 99% by weight of the total antiviral composition.

6. The antiviral composition for use according to claim 1, wherein the silver-containing substance is present in an amount between approximately 1% to approximately 35% by weight of the total antiviral composition.

7. The antiviral composition for use according to claim 1, wherein the unripe fig is present in an amount between approximately 65% and approximately 99% by weight of the total antiviral composition.

8. The antiviral composition for use according to claim 1, wherein the viral condition is selected from the group consisting of:
(a) warts,
(b) genital warts,
(c) shingles, and
(d) a disease caused by human papilloma virus.

## Patentansprüche

1. Antivirale Zusammensetzung zur Anwendung in der Behandlung eines viralen Zustands, umfassend:
(a) eine therapeutisch wirksame Menge einer Silber enthaltenden Substanz ausgewählt aus der Gruppe bestehend aus kolloidalem Silber, Silbernitrat und jede Kombination davon; und
(b) eine therapeutisch wirksame Menge an unreifer Feige;
(c) wobei die antivirale Zusammensetzung zur Behandlung des viralen Zustands in einem hiervon bedürftigen Subjekt geeignet ist.

2. Antivirale Zusammensetzung zur Anwendung gemäß Anspruch 1, worin die Silber enthaltende Substanz oder Silbernitrat in einer Menge zwischen etwa 1 und etwa 75 Gew.-% der gesamten antiviralen Zusammensetzung enthalten ist.

3. Antivirale Zusammensetzung zur Anwendung gemäß Anspruch 1, worin die unreife Feige in einer Menge zwischen etwa 25 und etwa 99 Gew.-% der gesamten antiviralen Zusammensetzung enthalten ist.

4. Antivirale Zusammensetzung zur Anwendung gemäß Anspruch 1, worin die Silber enthaltende Substanz in einer Menge zwischen etwa 1 und etwa 55 Gew.-% der gesamten antiviralen Zusammensetzung enthalten ist.

5. Antivirale Zusammensetzung zur Anwendung gemäß Anspruch 1, worin die unreife Feige in einer Menge zwischen etwa 45 und etwa 99 Gew.-% der gesamten antiviralen Zusammensetzung enthalten ist.

6. Antivirale Zusammensetzung zur Anwendung gemäß Anspruch 1, worin die Silber enthaltende Substanz in einer Menge zwischen etwa 1 und etwa 35 Gew.-% der gesamten antiviralen Zusammensetzung enthalten ist.

7. Antivirale Zusammensetzung zur Anwendung gemäß Anspruch 1, worin die unreife Feige in einer Menge zwischen etwa 65 und etwa 99 Gew.-% der gesamten antiviralen Zusammensetzung enthalten ist.

8. Antivirale Zusammensetzung zur Anwendung gemäß Anspruch 1, wobei der virale Zustand ausgewählt ist aus der Gruppe bestehend aus:
(a) Warzen,
(b) Genitalwarzen,
(c) Herpes Zoster, und
(d) einer Erkrankung verursacht durch das humane Papillomavirus.

## Revendications

1. Composition antivirale pour l'utilisation dans le traitement d'un état viral, comprenant:
(a) une quantité thérapeutiquement efficace d'une substance contenant de l'argent, choisie dans le groupe constitué par de l'argent colloïdal, du nitrate d'argent et toute combinaison de ceux-ci; et
(b) une quantité thérapeutiquement efficace de figue non mûre;
(c) dans laquelle la composition antivirale se prête au traitement de l'état viral chez un sujet en ayant besoin.

2. Composition antivirale pour l'utilisation selon la revendication 1, dans laquelle la substance contenant de l'argent ou le nitrate d'argent est présent(e) en une quantité comprise entre environ 1 % et environ 75 % en poids de l'ensemble de la composition antivirale.

3. Composition antivirale pour l'utilisation selon la revendication 1, dans laquelle la figue non mûre est présente en une quantité comprise entre environ 25 % et environ 99 % en poids de l'ensemble de la composition antivirale.

4. Composition antivirale pour l'utilisation selon la revendication 1, dans laquelle la substance contenant de l'argent est présente en une quantité comprise entre environ 1 % et environ 55 % en poids de l'ensemble de la composition antivirale.

5. Composition antivirale pour l'utilisation selon la revendication 1, dans laquelle la figue non mûre est présente en une quantité comprise entre environ 45 % et environ 99 % en poids de l'ensemble de la composition antivirale.

6. Composition antivirale pour l'utilisation selon la revendication 1, dans laquelle la substance contenant de l'argent est présente en une quantité comprise entre environ 1 % et environ 35 % en poids de l'ensemble de la composition antivirale.

7. Composition antivirale pour l'utilisation selon la revendication 1, dans laquelle la figue non mûre est présente en une quantité comprise entre environ 65 % et environ 99 % en poids de l'ensemble de la composition antivirale.

8. Composition antivirale pour l'utilisation selon la revendication 1, dans laquelle l'état viral est choisi dans le groupe constitué par:
(a) les verrues,
(b) les verrues génitales
(c) le zona et
(d) une maladie provoquée par le virus du papillome humain.
